# EUROPÄISCHE PATENTANMELDUNG

(11) **EP 0 643 949 A1**
(43) Veröffentlichungstag der Anmeldung: **22.03.1995**
(21) Anmeldenummer: 94114595.5
(22) Anmeldetag: 16.09.1994
(51) Int. Cl.: A61C 15/04, A61C 5/12, B65H 49/18

(54) **Fadenspendervorrichtung**

(30) Priorität: 18.09.1993 DE 9314140 U
(71) Anmelder: Beutter, Frank, D-70192 Stuttgart (DE)
(72) Erfinder: Beutter, Frank, D-70192 Stuttgart (DE)
(74) Vertreter: Patentanwälte Bartels, Held und Partner

(57) **Zusammenfassung**

Die Erfindung betrifft eine Fadenspendervorrichtung, insbesondere für einen Retraktionsfaden 10, der in vorgebbaren Längen von einer in einem Gehäuse 12 drehbar gelagerten Vorratsrolle 14 abwickelbar ist, der beim Austritt aus dem Gehäuse 12 mittels einer Schneidvorrichtung 18 durchtrennbar ist und der für den Austritt mittels einer Antriebseinrichtung 20 transportierbar, die mindestens eine Antriebsrolle 22 aufweist, im Gehäuse 12 geführt ist. Dadurch, daß die erste Antriebsrolle 22 mit einer zweiten Antriebsrolle 24 und diese wiederum mit einer dritten Antriebsrolle 26 zusammenwirkt und daß für das Zusammenwirken der Antriebsrollen 22, 24, 26 diese mit Zahnkränzen 28, 30, 32 versehen sind, die von der ersten 22 zu der dritten 26 Antriebsrolle in Reihe hintereinander angeordnet in Eingriff miteinander stehen, ist eine Fadenspendervorrichtung geschaffen, die eine einfache schnelle Einhandbedienung zuläßt und die einen sauberen, keimfreien sowie hemmfreien Transport des Behandlungsfadens erlaubt, der darüber hinaus in der Länge genau dosierbar ist.

## Beschreibung

Die Erfindung betrifft eine Fadenspendervorrichtung, insbesondere für einen Retraktionsfaden, der in vorgebbaren Längen von einer in einem Gehäuse drehbar gelagerten Vorratsrolle abwickelbar ist, der beim Austritt aus dem Gehäuse mittels einer Schneidvorrichtung durchtrennbar ist und der für den Austritt mittels einer Antriebseinrichtung transportierbar, die mindestens eine Antriebsrolle aufweist, im Gehäuse geführt ist. Als Retraktionsfäden kommen heute in der Regel gedrehte, geflochtene aber auch gewirkte, gestrickte und gehäkelte Baumwollfäden, die aus mehreren Filamenten bestehen, zur Anwendung. Dahingehende Retraktions-Kordfäden für die Retraktion von Zahnfleisch, die auch mit einer Retraktionslösung tränkbar sind und die das Versehen der Zähne mit Zahnkronen erleichtern, sind beispielhaft in der EP 0 131 360 beschrieben.

In der Vergangenheit war es ausschließlich üblich, dahingehende Retraktionsfäden in flaschenförmigen Behältern anzubieten und der darin lose liegende Faden mußte durch eine mit einem Kipphebelverschluß versehene Öffnung von Hand nach außen gezogen werden, um dann mit einer Schere abgeschnitten für die Verwendung beim Patienten zur Verfügung zu stehen. Häufig war der Faden hierbei meist nach wenigen Zentimetern, die entnommen wurden, in der Flasche verknotet und "lief" nicht mehr. Ferner benötigte man zwei Hände zur Entnahme und zum Abschneiden mit einer Schere. Gegebenenfalls mußte der dann verknotete Faden ganz entnommen und wieder entflochten werden, wodurch, abgesehen von dem Zeitaufwand, die Sterilität des Fadens verloren ging.

Um diese Nachteile zu vermeiden, ist bei einer gattungsgemäßen Vorrichtung nach der EP 0 166 883 B1 schon vorgeschlagen worden, Behandlungsfäden in wählbaren Abschnitten von einer in einem Gehäuse verdrehbar gehaltenen Vorratsrolle abzuwickeln, wobei der Behandlungsfaden in dem Gehäuse geführt und mittels einer verdrehbaren Antriebsrolle transportierbar ist, die aus dem Gehäuse umfangseitig, zumindest teilweise, herausragt und von einer Bedienperson von Hand unmittelbar antreibbar ist. Auf der der Antriebsrolle unmittelbar gegenüberliegenden Seite ist ein ebenfalls von Hand betätigbares Druckstück vorhanden, das mit einem Schneidmesser verbunden und entgegen der Kraft einer Feder den Durchtrennvorgang für den Faden durchführt, der zwischen der Antriebsrolle und einer der Umfangsform der Antriebsrolle angepaßten, feststehenden Führungsfläche hindurchführbar ist. Zwar läßt sich diese Vorrichtung mit nur einer Hand bedienen; es ist jedoch ein Zweifingerbetrieb (Zeigefinger und Daumen) notwendig, um zeitlich hintereinander zuerst die Antriebsrolle für den Austritt des Fadens aus dem Gehäuse zu bewegen und dann anschließend durch Betätigen des Druckstückes mit dem Daumen den Abschneidvorgang vorzunehmen. Neben einer etwas umständlichen Handhabung ragt darüber hinaus auch die Antriebsrolle aus dem Gehäuse hervor und kommt mit der Umgebung ebenso wie mit der Hand der Bedienperson in Berührung und anschließend mit dem Faden, so daß das Keimfreimachen der Vorrichtung und das Sterilhalten des Fadens kaum möglich ist. Da die Antriebsrolle längs ihres mittigen Außenumfanges mit einem Andrückring versehen ist, lassen sich mit der bekannten Vorrichtung sehr gut Matritzenbänder und Folien transportieren, jedoch keine Fäden, insbesondere keine Retraktionsfäden, da diese durch den Andrückring gestaucht in ihrem Weitertransport aus der Vorrichtung heraus behindert werden.

Ausgehend von diesem Stand der Technik liegt der Erfindung die Aufgabe zugrunde, eine Fadenspendervorrichtung zu schaffen, die eine einfache, schnelle Einhandbedienung zuläßt und die einen sauberen, keimfreien sowie hemmfreien Transport des Behandlungsfadens erlaubt, der darüber hinaus in der Länge genau dosierbar ist. Eine dahingehende Aufgabe löst eine Fadenspendervorrichtung mit den Merkmalen des Anspruches 1.

Dadurch, daß gemäß dem kennzeichnenden Teil des Anspruches 1 die erste Antriebsrolle mit einer zweiten Antriebsrolle und diese wiederum mit einer dritten Antriebsrolle zusammenwirkt und daß für das Zusammenwirken der Antriebsrollen diese mit Zahnkränzen versehen sind, die von der ersten zu der dritten Antriebsrolle in Reihe hintereinander angeordnet in Eingriff miteinander stehen, ist eine Art Transportgetriebe für den Faden verwirklicht, der einen sicheren, sauberen und störungsfreien Transport gewährleistet. Da alle Antriebsrollen in Reihe hintereinander miteinander in Eingriff stehen, läßt sich von einer zentralen Stelle aus der Transportvorgang für den Faden vornehmen, der von dieser zentralen Stelle aus auch abschneidbar ist. Es ist mithin nicht mehr notwendig, die Antriebsrollen für einen direkten Antrieb von Hand aus dem Gehäuse herauszuführen, und die Sterilität des Fadens ist nicht beeinträchtigt. Mit dem Antriebsgetriebe in Form der Antriebsrollen ist eine sichere Zwangsführung für den Faden gegeben, so daß es innerhalb der Vorrichtung zu keinen Aufstauchvorgängen des flexiblen Fadens kommt, welche den Fadentransport in negativer Weise beeinträchtigen könnten.

Bei einer bevorzugten Ausführungsform der erfindungsgemäßen Fadenspendervorrichtung weist die Antriebseinrichtung eine Handbetätigung mit einer Klinke auf, die beide entgegen einer Federkraft im Gehäuse längsverfahrbar geführt sind, wobei die Klinke mit einer der ersten Antriebsrolle zugeordneten Verzahnung in Eingriff steht, die koaxial zu dem Zahnkranz dieser Antriebsrolle angeordnet ist. In Abhängigkeit von der Verzahnung und den Zähnen des Zahnkranzes läßt sich mithin über die Antriebseinrichtung mit ihrer Handbetätigung taktweise in kleinen Schritten eine vorgebbare Fadenlänge rasch einstellen, die dann für die Behandlung zur Verfügung steht.

Bei einer weiteren bevorzugten Ausführungsform der erfindungsgemäßen Fadenspendervorrichtung ist die erste Antriebsrolle gegenüber einer feststehenden Führungsplatte für den Faden drehbar gelagert und wirkt mit einer elastisch nachgiebigen Raste zusammen, so daß eine ungewollte Rückstellbewegung für die erste Antriebsrolle vermieden ist und der Faden im Bereich des Austrittes aus dem Gehäuse nicht in das Gehäuse zurückfallen kann.

Bei einer weiteren besonders bevorzugten Ausführungsform der erfindungsgemäßen Fadenspendervorrichtung durchgreift die Achse der ersten Antriebsrolle die Führungsplatte, wobei auf der Führungsplatte ein Längsführungsstück für den Faden vorgesehen ist, das mit einer vorstehenden Führungsnase den Zuführbereich für den Faden zwischen den beiden anderen Antriebsrollen bildet. Hierdurch läßt sich der Faden genau definiert in den Zugriffsbereich zwischen den beiden Antriebsrollen für einen Weitertransport desselben in Richtung des Austritts einfädeln.

Bei einer weiteren bevorzugten Ausführungsform der erfindungsgemäßen Fadenspendervorrichtung sind die zweite und die dritte Antriebsrolle im Durchmesser und im Hinblick auf ihre Zahnkränze gleich ausgebildet, die einen kleineren Zahnkranzdurchmesser aufweisen als der Zahnkranz der ersten Antriebsrolle. Auch hierdurch ist gewährleistet, daß mit dem vorgesehenen Klinkentrieb der Antriebseinrichtung es zu einem optimalen Übersetzungsverhältnis kommt, so daß ein rascher, stetiger Vorschub des Fadens außerhalb der Vorrichtung gewährleistet ist.

Bei einer weiteren bevorzugten Ausführungsform der erfindungsgemäßen Fadenspendervorrichtung weist wahlweise die zweite oder die dritte oder sowohl die zweite als auch die dritte, vorzugsweise jedoch nur die zweite Antriebsrolle eine umfangseitig verlaufende Führungsrille auf, in die, zumindest teilweise, der Faden für seinen Weitertransport eingreift. Aufgrund dieser Ausgestaltung ist gewährleistet, daß der Faden in seiner Transportrichtung verbleibt und beispielsweise nicht längs den planen Außenumfangsflächen der Antriebsrollen abrutschen kann, was den hemmungsfreien Betrieb beeinträchtigen könnte.

Bei einer weiteren besonders bevorzugten Ausführungsform der erfindungsgemäßen Fadenspendervorrichtung ist zwischen dem Austritt aus dem Gehäuse und der zweiten und dritten Antriebsrolle eine Einlaufhilfe für den Faden angeordnet, die auslaufseitig in die Öffnung einer Gegenhalteplatte mündet, die in Fadentransportrichtung vor dem Austritt angeordnet ist und die eine Führungsfläche für ein längs dieser Fläche führbares Schneidmesser aufweist, das bei Betätigung die Öffnung überstreicht. Aufgrund dieser Anordnung ist die weitere Führung außerhalb des Eingriffsbereiches zwischen der zweiten und dritten Antriebsrolle sicher gewährleistet, so daß ein kontinuierlicher Austritt aus der Vorrichtung problemlos vonstatten geht und, selbst wenn der Faden beim Abschneiden an seinem verbleibenden Ende im Gehäuse sich auffächern sollte, ist ein nachfolgender Austrag desselben mittels der Transporteinrichtung problemlos möglich. Dank der Einlaufhilfe kann auch bei einem Wechsel der Vorratsrolle gegen eine solche mit einem neuen Faden der Einfädelvorgang für den Faden innerhalb der Vorrichtung rasch und fehlerfrei erfolgen.

Bei einer weiteren bevorzugten Ausführungsform der erfindungsgemäßen Fadenspendervorrichtung weist das Schneidmesser an seiner der Gegenhalteplatte zugeordneten Seite eine quer zu seiner Verfahrrichtung schräg verlaufende Schneide auf, wobei das der Schneide abgekehrte Ende des Schneidmessers mit der Handbetätigung fest verbunden ist. Hierdurch läßt sich mit der Handbetätigung also nicht nur der Vortrieb des Fadens, sondern bei entsprechender Betätigung auch von zentraler Stelle aus der Abschneidvorgang bewerkstelligen. Da die Schneide des Schneidmessers schräg verläuft, ist ein sauberer Anschnitt des Fadens gewährleistet und die Gefahr, daß sich dieser in seine Einzelfilamente an seinem freien Ende auflöst, ist vermieden.

Bei einer weiteren besonders bevorzugten Ausführungsform der erfindungsgemäßen Fadenspendervorrichtung ist das Gehäuse aus zwei Gehäusehälften, vorzugsweise aus durchsichtigem Kunststoffmaterial, gebildet, die miteinander lösbar verrastet sind, wobei eine Gehäusehälfte als Deckel ausgebildet von der anderen Gehäusehälfte abnehmbar ist, die im wesentlichen alle Funktionsteile der Vorrichtung bleibend aufnimmt. Aufgrund dieser Ausgestaltung läßt sich bei aufgebrauchtem Faden von der Vorratsrolle diese schnell und einfach gegen eine neue, mit Faden austauschen. Die erfindungsgemäße Fadenspendervorrichtung stellt also keinen Wegwerfartikel mehr dar, sondern bildet über das angesprochene Gehäuse eine Art Nachfüllbehälter aus, der sich beliebig oft mit neuem Fadenmaterial bestücken läßt.

Im folgenden ist die erfindungsgemäße Fadenspendervorrichtung anhand der Zeichnung näher erläutert.

Es zeigen:
- Fig.1: eine Draufsicht auf die geöffnete Fadenspendervorrichtung;
- Fig.2: in der Art einer Explosionszeichnung eine perspektivische Darstellung auf die Fadenspendervorrichtung gemäß der Fig.1;
- Fig.3: eine Draufsicht auf das Innere des Gehäusedeckels der Vorrichtung;
- Fig.4: in der Art einer Explosionszeichnung eine perspektivische Darstellung des Gehäusedeckels nach der Fig.3;
- Fig.5 und 6: in Draufsicht die Transport- und die Abschneidestellung für die Fadenspendervorrichtung nach den vorangegangenen Figuren.

Die erfindungsgemäße Fadenspendervorrichtung dient insbesondere dem Transport eines Retraktionsfadens 10, wie er in der Zahnmedizin heute üblich ist. Mit der Vorrichtung lassen sich jedoch auch andere Fäden, Nähfäden od.dgl., sowie Zahnseide durchaus transportieren. Der Retraktionsfaden 10 ist in vorgebbaren Längen von einer in einem Gehäuse 12 der Vorrichtung drehbar gelagerten Vorratsrolle 14 abwickelbar. Beim Austritt aus einer Austrittsstelle 16 des Gehäuses 12 ist der Faden 10 mittels einer als Ganzes mit 18 bezeichneten Schneidvorrichtung durchtrennbar und für den Austritt mittels einer als Ganzes mit 20 bezeichneten Antriebseinrichtung transportierbar.

Die Antriebseinrichtung 20 weist drei Antriebsrollen 22, 24 und 26 auf, wobei die erste Antriebsrolle 22 mit einer zweiten Antriebsrolle 24 und diese wiederum mit einer dritten Antriebsrolle 26 zusammenwirkt, wobei für das Zusammenwirken der Antriebsrollen 22,24,26 diese mit Zahnkränzen 28,30,32 versehen sind, die von der ersten 22 zu der dritten 26 Antriebsrolle in Reihe hintereinander angeordnet in Eingriff miteinander stehen. Die Antriebseinrichtung 20 weist des weiteren eine Handbetätigung 34 mit einer Klinke 36 auf, die beide entgegen der Federkraft einer Druckfeder 38 im Gehäuse 12 längsverfahrbar geführt sind, wobei das eine Ende der Druckfeder 38 sich an einer Anschlagplatte 40 im Gehäuse 12 abstützt, wohingegen das andere Ende im Inneren der als Hohlkammer ausgebildeten Handbetätigung 34 an dieser anliegt. Für das Längsverfahren der Handbetätigung 34 in dem Gehäuse 12 weist diese in Verfahrrichtung gesehen parallel zueinander verlaufende Seitenflächen auf, die in durch Gehäuseteile gebildeten Längsführungen geführt sind.

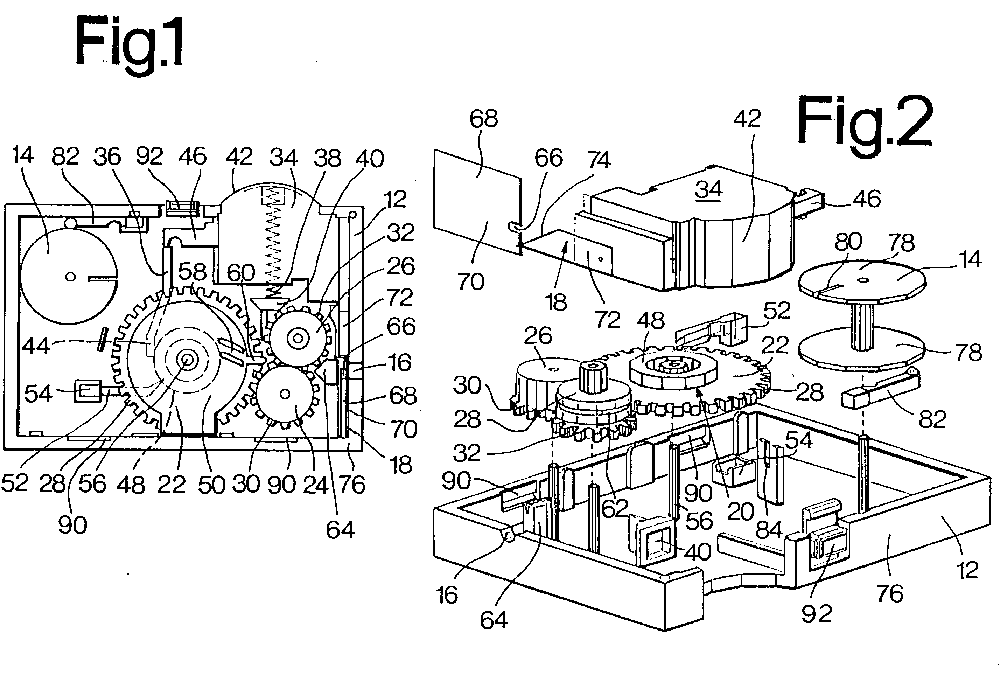

Sowohl die Klinke 36 als auch die Handbetätigung 34 sind vorzugsweise einstückig aus einem Kunststoffmaterial gebildet. Für die Anlage mit dem Daumen weist die Handbetätigung 34 eine der Daumenform angepaßte konvexe Wölbung mit einer Anlagefläche 42 auf. Die Klinke 36 weist ein freies abgekröpftes Ende 44 auf und das andere Ende ist über ein Mittelstück 46 einstückig mit der Handbetätigung 34 verbunden, wobei die Klinke 36 in Verfahrrichtung gesehen senkrecht auf dem Mittelstück 46 angeordnet ist. Wie insbesondere die Fig.1 zeigt, ist die Klinke 36 mit ihrem abgekröpften Ende 44 mit einer der ersten Antriebsrolle 22 zugeordneten Verzahnung 48 in Eingriff stehend, die koaxial zum Zahnkranz 28 dieser Antriebsrolle 22 angeordnet ist. Die Verzahnung 48, die in Blickrichtung auf die Fig.1 gesehen oberhalb des Zahnkranzes 28 angeordnet ist, kann mit Zähnen, wie bei den Zahnkränzen 28,30 und 32, versehen sein; die Verzahnung kann jedoch auch, wie in den Fig.1 und 2 dargestellt, im Sinne eines Vieleckes aus Vorsprüngen gebildet sein, an denen jeweils die Klinke 36 mit ihrem vorderen Ende 44 anliegt.

Die erste Antriebsrolle 22 ist gegenüber einer feststehenden Führungsplatte 50, deren Außenkontur der ersten Antriebsrolle 22 nachempfunden ist, drehbar gelagert, wobei die Verzahnung 48 mit einer elastisch nachgiebigen Raste 52 zusammenwirkt, die an ihrem anderen Ende wiederum in einer Ausnehmung 54 des Gehäuses 12 in seinem Inneren aufgenommen ist. Die drehfest innerhalb des Gehäuses 12 aufgenommene Achse 56 der ersten Antriebsrolle 22 durchgreift die Führungsplatte 50 und auf der Führungsplatte 50 ist ein Längsführungsstück 58 für den Faden 10 vorgesehen, das mit einer vorstehenden Führungsnase 60 den Zuführbereich für den Faden 10 zwischen den beiden anderen Antriebsrollen 24 und 26 bildet. In der Darstellung nach der Fig.1 gesehen ist also die fest mit dem Gehäuse 12 verbundene Führungsplatte 50 oberhalb der ersten Antriebsrolle 22 mit ihren diversen Verzahnungen angeordnet.

Die zweite 24 und die dritte 26 Antriebsrolle sind im Durchmesser und im Hinblick auf ihre Zahnkränze 30,32 gleich ausgebildet, die jeweils einen kleineren Zahnkranzdurchmesser aufweisen als der Zahnkranz 28 der ersten Antriebsrolle 22. Wahlweise weist die zweite 24 oder die dritte 26 oder sowohl die zweite 24 als auch die dritte 26, nach der Darstellung nach Fig.2 jedoch die dritte Antriebsrolle 26 eine umfangseitig verlaufende Führungsrille 62 auf, in die zumindest teilweise der Faden 10 für seinen Weitertransport eingreift.

Zwischen dem Austritt über die Austrittsstelle 16 aus dem Gehäuse 12 und der zweiten 24 und dritten 26 Antriebsrolle ist eine trichterförmige Einlaufhilfe 64 für den Faden 10 angeordnet, die auslaufseitig in die Öffnung 66 einer Gegenhalteplatte 68 mündet, die in Fadentransportrichtung vor dem Austritt im Gehäuse 12 angeordnet ist, wobei die Gegenhalteplatte 68 eine der Gehäuseinnenseite zugewandte Führungsfläche 70 aufweist, für ein längs dieser Fläche 70 führbares Schneidmesser 72, das mit der Handbetätigung 34 fest verbunden ist und bei deren vollständiger Betätigung die Öffnung 66 von dem Schneidmesser 72 überstrichen wird. Die Gegenhalteplatte 68 ist also mit einem vorgebbaren axialen Abstand gegenüber der zugeordneten Gehäuseinnenwand derart zurückversetzt, daß in den freigegebenen Spalt das Schneidmesser 72 für einen Abschneidvorgang des Fadens 10 eindringen kann. Wie insbesondere die Fig.2 weiter zeigt, weist das Schneidmesser 72 an seiner der Gegenhalteplatte 68 zugeordneten Seite eine quer zu seiner Verfahrrichtung schräg verlaufende Schneide 74 auf, wobei das der Schneide 74 abgekehrte Ende des Schneidmessers 72 mit einer Seitenfläche der Handbetätigung 34 fest verbunden ist.

Nicht nur die erste Antriebsrolle 22 ist über die ihr zugeordnete Achse 56 drehbar mit dem Gehäuse 12 verbunden, sondern vielmehr über entsprechende und nicht näher bezeichnete Achsen auch die Vorratsrolle 14 sowie die zweite 24 und dritte 26 Antriebsrolle, wobei alle Achsen mit nur einer Gehäusehälfte, hier die untere Gehäusehälfte 76, fest verbunden sind oder, sofern das Gehäuse 12 aus einem Kunststoff-Spritzmaterial besteht, Teile desselben sind. Zumindest die Achse der zweiten 24 und der dritten 26 Antriebsrolle können auch etwas nachgiebig ausgebildet sein, so daß sie sich von Hand aus ihrer Richtung kippen lassen, was bei geöffneter Vorrichtung den Einfädelvorgang zwischen die genannten Rollen erleichtert. Die Vorratsrolle 14, die zwischen ihren endseitigen Deckelplatten 78 den Faden 10 bevorratet, der mit seinem einen Ende in einem Schlitz 80 der oberen Deckelplatte 78 (nicht dargestellt) einklemmbar ist, wird über ihre untere Deckelplatte 78 mit einem Führungsdorn 82 in Anlage gebracht, der an seinem anderen Ende in einer entsprechenden Ausnehmung innerhalb des Gehäuses 12 festgelegt ist. Für einen korrekten Transport des Fadens innerhalb der Vorrichtung ist vor der ersten Antriebsrolle 22 und der Führungsplatte 50, die in der Fig.2 der Einfachheit halber nicht dargestellt ist, in Transportrichtung gesehen hinter der Vorratsrolle 14 ein Führungsschlitz 84 vorgesehen, in den der Faden 10 eingreift und der ein stegartiger Teil der unteren Gehäusehälfte 76 ist.

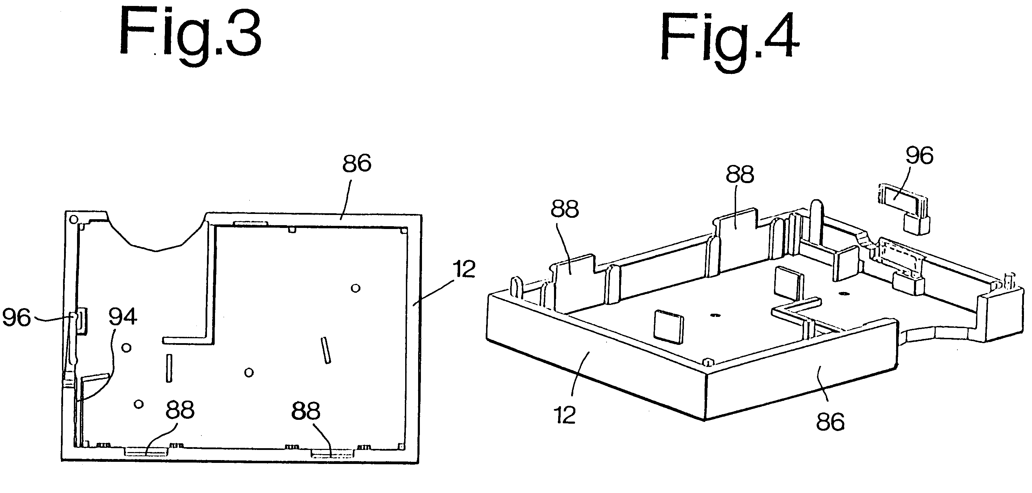

Wie bereits angesprochen besteht das Gehäuse 12 aus zwei Gehäusehälften, untere Gehäusehälfte 76 und obere Gehäusehälfte 86, vorzugsweise aus durchsichtigem, spritzbarem Kunststoffmaterial, die miteinander lösbar verrastet sind, wobei die obere Gehäusehälfte gemäß den Fig.3 und 4 als Deckel ausgebildet von der anderen unteren Gehäusehälfte 76 abnehmbar ist, die im wesentlichen alle vorangegangenen Funktionsteile der Vorrichtung bleibend bis auf einen etwaigen Austausch der Vorratsrolle 14 mit Faden 10 aufnimmt. Die obere Gehäusehälfte 86 weist an ihrer der Handbetätigung 34 abgekehrten Seite zwei randseitig überstehende Eingreifteile 88 auf, die in entsprechend geformte Ausnehmungen 90 (s.Fig.1 und 2) der unteren Gehäusehälfte 76 einsetzbar sind. Es ist dann eine Art Scharniergelenk verwirklicht und beim Zusammenklappen der beiden Gehäusehälften 76 und 86 miteinander kann eine Verrastung derselben miteinander über ein Kunststoff-Druckknopfteil 92 erfolgen. Des weiteren weist die obere Gehäusehälfte 86 an ihrer Seite, die der Handbetätigung 34 benachbart ist, einen Ausnehmungskanal 94 auf, in den ein Teil der Gegenhalteplatte 68 eingreifen kann. Neben den bereits beschriebenen Verrastungsmöglichkeiten für die Verbindung der Gehäusehälften 76 und 78 miteinander kann an derselben Seite, wo der Ausnehmungskanal 94 liegt, ein Federteil 96 vorgesehen sein, das mit einem axialen Überstand nachgiebig über die Innenseite der Gehäusehälfte 86 vorsteht und beim Verbinden der Gehäusehälften 76 und 86 miteinander kraftschlüssig rückverschwenkt wird und eine Haltekraft auf die miteinander verbundenen Gehäusehälften ausübt.

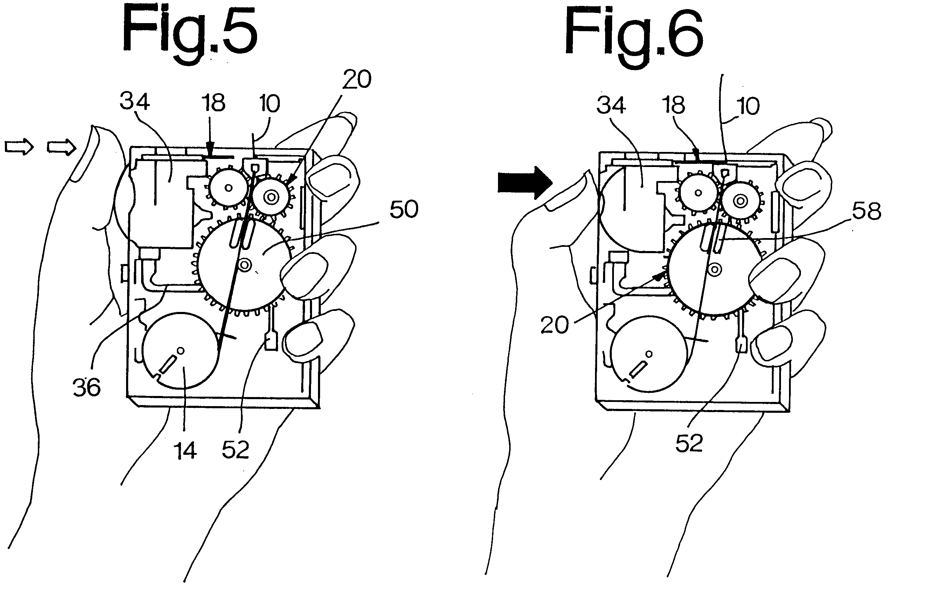

Anhand der Fig.5 und 6 sollen nunmehr noch näher die Funktionsweise der erfindungsgemäßen Fadenspendervorrichtung näher erläutert werden. Drückt die Bedienperson mit ihrem Daumen gemäß der Pfeilrichtung mehrmals leicht auf die Handbetätigung 34, schiebt jedes Mal die Klinke 36 mit ihrem abgekröpften Ende 44 über die Verzahnung 48 die erste Antriebsrolle 22 entgegen dem Uhrzeigersinn radial weiter. Hierbei wird eine Rückstellbewegung der ersten Antriebsrolle 22 über die Raste 52 vermieden. Nach jedem leichten Daumendruck wird die Handbetätigung 34 zusammen mit der Klinke 36 über die Druckfeder 38 wieder in ihre nicht betätigte Stellung rückbewegt. Durch das taktweise Ansteuern der ersten Antriebsrolle 22 wird über ihre Verzahnung 28 die zweite Verzahnung 30 und mithin die zweite Antriebsrolle 24 im Uhrzeigersinn bewegt und die mit der zweiten Antriebsrolle 24 in Eingriff über ihre Verzahnung 30 stehende dritte Antriebsrolle 26 wiederum entgegen dem Uhrzeigersinn. Dadurch, daß der Faden zwischen den beiden Antriebsrollen 24 und 26 und in der Führungsrille 62 der dritten Antriebsrolle 26 geführt ist, kommt es zu einer Zwangsführung mit der Folge, daß im Rhythmus der Bewegung aller Antriebsrollen 22,24,26 der Faden 10 über die Austrittsstelle 16 nach außen transportiert wird.

Betätigt man die Handbetätigung 34 beispielsweise zweimal kurz hintereinander, so wird die doppelte Menge an Faden 10 nach außen transportiert, als bei nur einem Drückvorgang; bei dreimaliger Betätigung der Handbetätigung 34 wird dann die dreifache Länge usw. gefördert, bis die Bedienperson die Fadenlänge nach außen transportiert hat, die sie eben benötigt. Bei diesem Transportvorgang wird zwar zusammen mit der Handbetätigung 34 gleichfalls das Schneidmesser 72 vor- und zurückbewegt; jedoch gelangt es nie so weit vor, als daß es den Faden 10 an- oder gar durchschneiden könnte. Erst wenn, wie dies in Fig.6 angedeutet ist, die Handbetätigung 34 vollständig entgegen der Kraft der Druckfeder 38 durchgedrückt ist, kommt es zu einem Abschneidvorgang und die Vorrichtung steht für einen erneuten Transportvorgang der Bedienperson nach Loslassen der Handbetätigung 34 zur Verfügung.

## Patentansprüche

1. Fadenspendervorrichtung, insbesondere für einen Retraktionsfaden (10), der in vorgebbaren Längen von einer in einem Gehäuse (12) drehbar gelagerten Vorratsrolle (14) abwickelbar ist, der beim Austritt aus dem Gehäuse (12) mittels einer Schneidvorrichtung (18) durchtrennbar ist und der für den Austritt mittels einer Antriebseinrichtung (20) transportierbar, die mindestens eine Antriebsrolle (22) aufweist, im Gehäuse (12) geführt ist, dadurch gekennzeichnet, daß die erste Antriebsrolle (22) mit einer zweiten Antriebsrolle (24) und diese wiederum mit einer dritten Antriebsrolle (26) zusammenwirkt und daß für das Zusammenwirken der Antriebsrollen (22,24,26) diese mit Zahnkränzen (28,30,32) versehen sind, die von der ersten (22) zu der dritten (26) Antriebsrolle in Reihe hintereinander angeordnet in Eingriff miteinander stehen.

2. Fadenspendervorrichtung nach Anspruch 1, dadurch gekennzeichnet, daß die Antriebseinrichtung eine Handbetätigung (34) mit einer Klinke (36) aufweist, die beide entgegen einer Federkraft im Gehäuse (12) längsverfahrbar geführt sind, und daß die Klinke (36) mit einer der ersten Antriebsrolle (22) zugeordneten Verzahnung (48) in Eingriff steht, die koaxial zu dem Zahnkranz (28) dieser Antriebsrolle (22) angeordnet ist.

3. Fadenspendervorrichtung nach Anspruch 2, dadurch gekennzeichnet, daß die erste Antriebsrolle (22) gegenüber einer feststehenden Führungsplatte (50) für den Faden (10) drehbar gelagert ist und mit einer elastisch nachgiebigen Raste (52)zusammenwirkt.

4. Fadenspendervorrichtung nach Anspruch 3, dadurch gekennzeichnet, daß die Achse (56) der ersten Antriebsrolle (22) die Führungsplatte (50) durchgreift und daß auf der Führungsplatte (50) ein Längsführungsstück (58) für den Faden (10) vorgesehen ist, das mit einer vorstehenden Führungsnase (60) den Zuführbereich für den Faden (10) zwischen den beiden anderen Antriebsrollen (24,26) bildet.

5. Fadenspendervorrichtung nach einem der Ansprüche 1 bis 4, dadurch gekennzeichnet, daß die zweite (24) und die dritte (26) Antriebsrolle im Durchmesser und im Hinblick auf ihre Zahnkränze (30,32) gleich ausgebildet sind, die einen kleineren Zahnkranzdurchmesser aufweisen als der Zahnkranz (28) der ersten Antriebsrolle (22).

6. Fadenspendervorrichtung nach einem der Ansprüche 1 bis 5, dadurch gekennzeichnet, daß wahlweise die zweite (24) oder die dritte (26) oder sowohl die zweite (24) als auch die dritte (26), vorzugsweise jedoch nur die zweite Antriebsrolle (26) eine umfangseitig verlaufende Führungsrille (62) aufweist, in die zumindest teilweise der Faden (10) für seinen Weitertransport eingreift.

7. Fadenspendervorrichtung nach einem der Ansprüche 1 bis 6, dadurch gekennzeichnet, daß zwischen dem Austritt aus dem Gehäuse (12) und der zweiten (24) und dritten (26) Antriebsrolle eine Einlaufhilfe (64) für den Faden (10) angeordnet ist, die auslaufseitig in die Öffnung (66) einer Gegenhalteplatte (68) mündet, die in Faden-Transportrichtung vor dem Austritt angeordnet ist und die eine Führungsfläche (70) für ein längs dieser Fläche (70) führbares Schneidmesser (72) aufweist, das bei Betätigung die Öffnung (66) überstreicht.

8. Fadenspendervorrichtung nach Anspruch 7, dadurch gekennzeichnet, daß das Schneidmesser (72) an seiner der Gegenhalteplatte (68) zugeordneten Seite eine quer zu seiner Verfahrrichtung schräg verlaufende Schneide (74) aufweist und daß das der Schneide (74) abgekehrte Ende des Schneidmessers (72) mit der Handbetätigung (34) fest verbunden ist.

9. Fadenspendervorrichtung nach einem der Ansprüche 1 bis 8, dadurch gekennzeichnet, daß das Gehäuse (12) aus zwei Gehäusehälften (76,86), vorzugsweise aus durchsichtigem Kunststoffmaterial, gebildet ist, die miteinander lösbar verrastet sind, und daß eine Gehäusehälfte (86) als Deckel ausgebildet von der anderen Gehäusehälfte (76)abnehmbar ist, die im wesentlichen alle Funktionsteile der Vorrichtung bleibend aufnimmt.
